# EUROPÄISCHE PATENTSCHRIFT

(11) **EP 1 566 448 B1**
(45) Veröffentlichungstag und Bekanntmachung des Hinweises auf die Patenterteilung: **25.03.2009**
(21) Anmeldenummer: 04025211.6
(22) Anmeldetag: 22.10.2004
(51) Int. Cl.: C12Q 1/25

(54) **Differenzialdiagnostik mit Hepcidin**
Differential diagnosis with Hepcidine
Diagnose différentielle avec la hepcidine

(30) Priorität: 22.10.2003 DE 10349124
(43) Veröffentlichungstag der Anmeldung: 24.08.2005
(73) Patentinhaber: F.HOFFMANN-LA ROCHE AG, 4070 Basel (CH); Roche Diagnostics GmbH, 68305 Mannheim (DE)
(72) Erfinder: Lehmann, Paul, 67549 Worms (DE); Roeddiger, Ralf, 69517 Gorxheimertal (DE)
(74) Vertreter: Dey, Michael

(56) Entgegenhaltungen:
- WO-A-20/04058044
- NEMETH ELIZABETA ET AL: "Hepcidin, a putative mediator of anemia of inflammation, is a type II acute-phase protein." BLOOD. 1 APR 2003, Bd. 101, Nr. 7, 1. April 2003 (2003-04-01), Seiten 2461-2463, XP002356755 ISSN: 0006-4971
- NICOLAS GAËL ET AL: "The gene encoding the iron regulatory peptide hepcidin is regulated by anemia, hypoxia, and inflammation." THE JOURNAL OF CLINICAL INVESTIGATION. OCT 2002, Bd. 110, Nr. 7, Oktober 2002 (2002-10), Seiten 1037-1044, XP002356756 ISSN: 0021-9738
- WEINSTEIN DAVID A ET AL: "Inappropriate expression of hepcidin is associated with iron refractory anemia: implications for the anemia of chronic disease." BLOOD. 15 NOV 2002, Bd. 100, Nr. 10, 15. November 2002 (2002-11-15), Seiten 3776-3781, XP002356757 ISSN: 0006-4971
- ROETTO ANTONELLA ET AL: "Mutant antimicrobial peptide hepcidin is associated with severe juvenile hemochromatosis." NATURE GENETICS. JAN 2003, Bd. 33, Nr. 1, Januar 2003 (2003-01), Seiten 21-22, XP002356759 ISSN: 1061-4036
- OKKA MEHMET ET AL: "Plasma homocysteine level and uveitis in Behçet's disease." THE ISRAEL MEDICAL ASSOCIATION JOURNAL : IMAJ. NOV 2002, Bd. 4, Nr. 11 Suppl, November 2002 (2002-11), Seiten 931-934, XP009058006 ISSN: 1565-1088
- THEUMA P ET AL: "Inflammation and emerging risk factors in diabetes mellitus and atherosclerosis" CURRENT DIABETES REPORTS 2003 UNITED KINGDOM, Bd. 3, Nr. 3, Juni 2003 (2003-06), Seiten 248-254, XP009058007 ISSN: 1534-4827

## Beschreibung

Die vorliegende Erfindung betrifft ein Verfahren zum Nachweis von entzündlichen chronischen Erkrankungen, nicht-entzündlichen chronischen Erkrankungen oder/und Akut-Phase-Reaktionen, umfassend die Bestimmung von Hepcidin und einem Akut-Phase-Protein oder/und einem Regulator der Synthese von Akut-Phase-Proteinen sowie ein Verfahren zum Nachweis von chronischen Erkankungen umfassend die Bestimmung von Hepcidin und Homocystein.

Chronische Erkrankungen sind weit verbreitet und umfassen eine Vielzahl von entzündlichen und nicht entzündlichen Erkrankungen verschiedenster Organe. Chronische Erkrankungen bedingen oft eine erhebliche Einschränkung der Lebensqualität der betroffenen Patienten. Deshalb ist es wünschenswert, schnellere und sicherere Diagnosemöglichkeiten bereitzustellen, um somit die Therapie und Betreuung von Patienten mit chronischen Erkrankungen zu optimieren.

Bei der Diagnose von Erkrankungen werden oftmals bestimmte physiologische Parameter, wie beispielsweise Gammaglobuline, Blutzuckerspiegel oder andere Blutwerte bestimmt. Die Unterscheidung bzw. Bestimmung von nicht-entzündlichen chronischen Erkrankungen, entzündlichen chronischen Erkrankungen oder/und Akut-Phase-Reaktionen ist jedoch problematisch. Für viele chronische Erkrankungen gibt es bis heute keine spezifischen Marker. Zur Diagnose chronisch entzündlicher Erkrankungen werden im Stand der Technik beispielsweise allgemeine Entzündungsmarker herangezogen. Problematisch hierbei ist allerdings, dass viele dieser Parameter nicht nur bei einer chronischen Entzündung sondern auch bei einer Akut-Phase-Reaktion, beispielsweise einer Infektion oder Verletzung bei einem Patienten erhöht bzw. verändert sind.

Somit besteht ein großer Bedarf an neuen Markern, die für diagnostische Fragestellungen im Zusammenhang mit chronischen Erkrankungen geeignet sind. Insbesondere besteht ein Bedarf an Markern, welche eine Unterscheidung von nicht-entzündlichen chronischen Erkrankungen, entzündlichen chronischen Erkrankungen oder/und Akut-Phase-Reaktionen ermöglichen.

Nemeth et al., (Blood., Band 101, Nummer 7 (2003)) offenbart eine Rolle von Hepcidin im Zusammenhang mit Entzündungen, Eisenstoffwechsel und Anämie.

Weinstein et al., (Blood., Band 100, Nummer 10 (2002)) beschreibt einen Zusammenhang zwischen Hepcidinexpression und Entzündungsstimuli. Ferner wird ein Zusammenhang zwischen Hepcidin und Anämie bei chronischen Erkrankungen allgemein sowie einer refraktären Anämie/Leberadenom im Besonderen beschrieben.

Nicolas et. al., (The Journal of Clinical Investigation, Band 110, Nummer 7 (2002)) beschreibt die Rolle von Hepcidin bei Erkrankungen im Zusammenhang mit Eisenüberladung und Eisenmangel. Ferner wird die Rolle von Hepcidin während eines Entzündungszustandes, der mit Störungen des Eisenstoffwechsels im Zusammenhang steht, diskutiert.

Roetto Antonello et. al., (Nature Genetics, Band 33, Nummer 1 (2003)) offenbart einen Zusammenhang von Hepcidin und der juvenilen Hämochromatose. Ferner wird eine Funktion von Hepcidin als Regulator der Eisenabsorption offenbart.

Morgenstern et. al., (Digestive Diseases and Sciences, Band 48, Nummer 10 (2003)) beschreibt eine Studie an Patienten mit entzündlichen Darmerkrankungen. Erhöhte Homocysteinwerte werden dabei auf verschiedenste Ursachen zurückgeführt.

Überraschenderweise wurde nun gefunden, dass Hepcidin in Kombination mit Homocystein oder/und einem Akut-Phase-Protein oder/und einem Regulator der Synthese von Akut-Phase-Proteinen als Marker in Verfahren zum Nachweis von entzündlichen chronischen Erkrankungen verwendet werden kann, insbesondere bei der Differenzialdiagnostik zum Nachweis von entzündlichen chronischen Erkrankungen oder/und nicht-entzündlichen chronischen Erkrankungen.

Hepcidin ist ein Peptid aus 20 (SEQ ID NO:1), 22 (SEQ ID NO:2) oder 25 (SEQ ID NO:3) Aminosäuren (Park et al., J. Biol. Chem. Vol. 276, Nr. 11, S. 7806-7810, 2001), welche von Hepcidinogen abgespalten werden. Hepcidin wird in der Leber produziert und spielt im Eisenstoffwechsel eine zentrale Rolle (Nicolas et al., Blood cells, Molecules and Diseases (2002) 29(3): 327-335).

Es wird gegenwärtig angenommen, dass Hepcidin die Eisenabgabe aus den Makrophagen an die erythropoietischen Vorläuferzellen reguliert, wobei ein erhöhter Hepcidinspiegel die Eisenabgabe hemmt. In Makrophagen bewirkt eine hohe Eisenkonzentration eine Erhöhung der induzierten Stickoxidsynthetase. Dies induziert die Cytokinproduktion, was wiederum chronische Entzündungen auslösen kann.

Somit ist es vorteilhafterweise möglich, chronische Erkrankungen, die beispielsweise durch einen Funktionseisenmangel charakterisiert sind, Hämochromatosen, Diabetes, rheumatoide Arthritis, Arteriosklerose, Tumoren oder Nierenversagen mit Hilfe des Markers Hepcidin zu diagnostizieren.

Hepcidin kann beispielsweise aus Serum, Plasma oder Urin bestimmt werden. Bevorzugt erfolgt eine Bestimmung aus Serum. Die Referenzwerte der Messgröße Hepcidin liegen beispielsweise im Bereich von etwa 0 bis 1000 ng/ml, insbesondere 100 bis 500 ng/ml. Ein besonders bevorzugter Referenzbereich ist etwa 200 bis 260 ng/ml. Als Grenzwert kann bei der Messung jeder beliebige Wert innerhalb des Referenzbereichs verwendet werden, beispielsweise ein Wert im Bereich von 0 bis 1000 ng/ml. Bevorzugt wird als Grenzwert ein Wert zwischen 100 und 500 ng/ml eingesetzt.

Die Bestimmung von Hepcidin kann beispielsweise mit dem Hepcidin Prohormone ELISA Test der Fima DRG, Marburg, Deutschland durchgeführt werden.

Eine besonders gute und sichere erfindungsgemäße Diagnosemöglichkeit ergibt sich, wenn neben Hepcidin zusätzlich ein oder mehrere Marker eingesetzt werden. Bevorzugt werden Marker bestimmt, welche zum Nachweis von entzündlichen chronischen Erkrankungen und/oder nicht entzündlichen chronischen Erkrankungen geeignet sind. Erfindungsgemäß verwendete Marker sind Akut-Phase-Proteine, Regulatoren der Synthese von Akut-Phase-Proteinen und Homocystein. Weitere bevorzugt verwendete Maker sind hs-CRP, SAA, C-reaktives Protein (CRP), Serum-Amyloid A (SAA), α₁-Anti-chymotrypsin, saures α₁-Glykoprotein, α₁-Antitrypsin, Haptoglobin, Fibrinogen, Komplement Komponente C3, Komplement Komponente C4, Coeruloplasmin, Interleukin 6 (IL-6), Leukemia inhibiting factor (LIF), Oncostatin M, Interleukin 11 (IL-11), Ciliary neurotropic factor (CNTF), Interleukin 1α (IL-1 α), Interleukin 1β (IL-1β), Tumornekrosefaktor-α (TNFα), Tumornekrosefak-β (TNFβ), Insulin, Fibroplastenwachstumsfaktor (FGF; fibroblast growth factor), Hepatocytenwachstumsfaktor, Transgrowth factor β (TGFβ ) oder/und Interferon. Darüber hinaus können auch weitere, im Fachgebiet verwendete Marker eingesetzt werden.

Durch die Verwendung von ein oder mehreren weiteren Markern neben Hepcidin kann eine genauere und sicherere diagnostische Abklärung einer Erkrankung gewährleistet werden. Dadurch können die psychischen und physischen Belastungen betroffener Patienten durch gezielte Therapiemaßnahmen noch schneller verringert bzw. beseitigt werden. Die Verwendung von mehreren Markern ist insbesondere dann von Vorteil, wenn bei einem Patienten neben einer chronischen Erkrankung eine oder mehrere weitere Erkrankungen vorliegen.

Bei manchen diagnostischen Fragestellungen ist es besonders wünschenswert, eine Unterscheidung zwischen dem Vorliegen einer nicht-entzündlichen oder/und entzündlichen chronischen Erkrankung zu treffen. Überraschenderweise wurde nun gefunden, dass dies durch eine Bestimmung der Marker Hepcidin und Homocystein möglich ist.

Somit betrifft die vorliegende Erfindung ein Verfahren zum Nachweis von chronischen Erkrankungen, umfassend die Bestimmung von
(i) Hepcidin und
(ii) Homocystein.

Mit dem erfindungsgemäßen Verfahren können beispielsweise entzündliche oder/und nicht-entzündliche chronische Erkrankungen nachgewiesen werden. Insbesondere ist es durch die Bestimmung von Hepcidin und Homocystein vorteilhaftweise möglich, zwischen dem Vorliegen einer nicht-entzündlichen chronischen Erkrankung oder/und einer entzündlichen chronischen Erkrankung zu unterscheiden bzw. eine eindeutige Zuordnung zu einer nicht-entzündlichen oder/und entzündlichen chronischen Erkrankung durchzuführen. Hierbei zeigt ein erhöhter Hepcidingehalt das Vorliegen einer entzündlich chronischen Erkrankung an, ein erhöhter Homocysteingehalt deutet auf eine nicht-entzündliche chronische Erkrankung hin.

Entzündliche chronische Erkrankungen, welche durch das erfindungsgemäße Verfahren nachgewiesen werden können, sind beispielsweise Hämatochromatosen, Diabetes, rheumatoide Arthritis, und andere rheumatische Erkrankungen, Arteriosklerose, Vaskulitis, Tumoren, Nierenversagen und systemischer Lupus erythematodes. Nicht-entzündliche chronische Erkrankungen, welche durch das erfindungsgemäße Verfahren nachgewiesen werden können, sind beispielsweise Vitaminmangelzustände, insbesondere B6-, B12- und Folatmangelzustände, Pellagra, fumikuläre Spinalerkrankung, Pseudoencephalitis, hemodegenerative Erkrankungen, wie z.B. Alzheimer und vaskuläre Erkrankungen wie z.B. koronare Herzkrankheit oder periphere arterielle Verschlusskrankheit.

Die Bestimmung von Hepcidin bzw. die Grenzwerte sind wie zuvor beschrieben.

Homocystein kann beispielsweise aus Serum, Plasma oder Urin bestimmt werden. Bevorzugt erfolgt eine Bestimmung aus Serum. Die Referenzwerte der Messgröße Homocystein liegen bevorzugt im Bereich von etwa 3 bis 18 µMol/l, bevorzugt etwa 5 bis 15 µMol/l, insbesondere etwa 15 µMol/l. Als Grenzwert kann bei der Messung jeder beliebige Wert innerhalb des Referenzbereichs verwendet werden, beispielsweise 12, 13, 14 oder 15 µMol/l. Besonders bevorzugt wird als Grenzwert etwa 15 µMol/l eingesetzt.

In dieser Ausführungsform des erfindungsgemäßen Verfahrens erfolgt bevorzugt eine Klassifizierung in die Gruppen:
a) keine chronische Erkrankung,
b) entzündliche chronische Erkrankung,
c) nicht-entzündliche chronische Erkrankung und
d) sowohl entzündliche chronische Erkrankung als auch nicht-entzündliche chronische Erkrankung.

Die folgende Darstellung veranschaulicht ein Klassifizierungsmodell, ausgehend von der Bestimmung von Hepcidin und Homocystein.

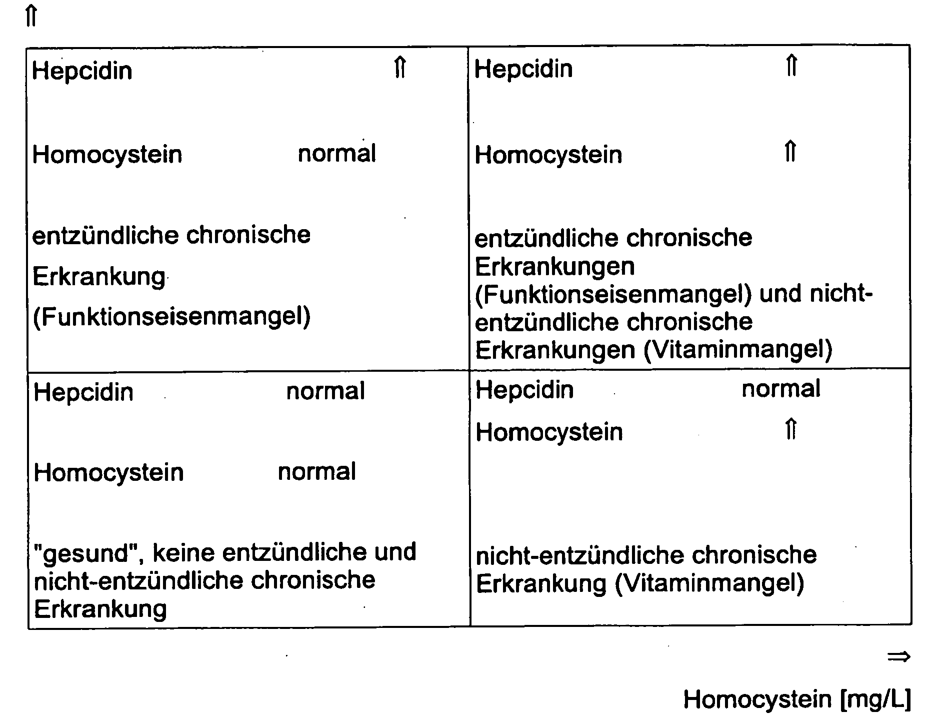

Auch in dieser Ausführungsform des erfindungsgemäßen Verfahrens können neben Hepcidin und Homocystein zusätzlich ein oder mehrere Marker verwendet werden. Geeignete Marker sind oben angeführt.

Im klinischen Alltag ergeben sich häufig Fragestellungen, wobei eine Diagnostik bzw. eine Unterscheidung zwischen dem Vorliegen von entzündlichen chronischen Erkrankungen oder/und temporären Akut-Phase-Reaktionen wünschenswert ist, welche oft ähnliche Symptome aufweisen. Die Verwendung von allgemeinen Entzündungsmarkern ist hierbei meist nicht zufriedenstellend, da diese sowohl bei temporären Akut-Phase-Reaktionen als auch chronischen entzündlichen Erkrankungen erhöht bzw. verändert sind. Es wurde nun gefunden, dass durch eine Bestimmung von Hepcidin und einem Akut-Phase-Protein oder/und einem Regulator der Synthese von Akut-Phase-Proteinen eine Unterscheidung von entzündlichen chronischen Erkrankungen und temporären Akut-Phase-Reaktionen möglich ist.

In einem weiteren Aspekt betrifft die vorliegende Erfindung somit ein Verfahren zum Nachweis von entzündlichen chronischen Erkrankungen oder/und Akut-Phase-Reaktionen, umfassend die Bestimmung von
(i) Hepcidin und
(iii) einem Akut-Phase-Protein oder/und einem Regulator der Synthese von Akut-Phase-Proteinen.

Beispiele für Akut-Phase-Proteine, welche im erfindungsgemäßen Verfahren bevorzugt bestimmt werden, sind beispielsweise C-reaktives Protein (CRP), Serumamyloid A (SAA), α-1-Antichymotrypsin, saures α1-Glycoprotein, α1-Antitrypsin, Haptoglobin, Fibrinogen, die Komplementkomponente C3, die Komplementkomponente C4 oder/und Coerroloplasmin. Beispiele für Regulatoren der Synthese von Akut-Phase-Proteinen sind beispielsweise Interleukin-6, der Leukämieinhibitionsfaktor (LEF), Oncostatin M, Interleukin 11, der ciliare neurotropische Faktor (CNTF), Interleukin 1α (IL-1 α), Interleukin 1β (IL-1β), der Tumornekrosefaktor α (TNF α), Der Tumornekrosefaktor β (TNFβ), Insulin, der Fibroblastenwachstumsfaktor, der Hepatocytenwachstumsfaktor, der Transwachstumsfaktor β (TGFβ) oder/und Interferon. Bevorzugt werden CRP, SAA, IL6, IL1 oder/und TNF-α bestimmt. Besonders bevorzugt wird im erfindungsgemäßen Verfahren das Akut-Phase-Protein C-reaktives Protein bestimmt.

Die Referenzwerte und Bestimmungsmöglichkeiten von Hepcidin sind hierbei wie oben beschrieben.

Geeignete Referenzwerte der Messgröße C-reaktives Protein liegen beispielsweise im Bereich von 7 bis 13 mg/l, bevorzugt etwa 8 bis 12 mg/l, besonders bevorzugt etwa 9 bis 11 mg/l, insbesondere etwa 10 mg/l. Als Grenzwert kann bei der Messung jeder beliebige Wert innerhalb des Referenzbereichs verwendet werden, beispielsweise 9, 10 oder 11 mg/l. Besonders bevorzugt wird als Grenzwert etwa 10 mg/l eingesetzt.

Bei dieser Ausführungsform des erfindungsgemäßen Verfahrens erfolgt bevorzugt eine Klassifizierung in die folgenden Gruppen:
a) keine Akut-Phase-Reaktion, keine entzündliche chronische Erkrankung,
b) entzündliche chronische Erkrankung mit Akut-Phase-Reaktion, und
c) Akut-Phase-Reaktion, keine entzündliche chronische Erkrankung und
d) entzündliche chronische Erkrankung, keine Akut-Phase-Reaktion.

Die Bestimmung von Hepcidin und einem Akut-Phase-Protein oder/und einem Regulator der Synthese von Akut-Phase-Proteinen ermöglicht es vorteilhafterweise, zwischen dem Vorliegen einer entzündlichen chronischen Erkrankung oder/und einer temporären Akut-Phase-Reaktion zu unterscheiden. Hierbei ist ein erhöhter Hepcidin-Gehalt ein Hinweis auf das Vorliegen einer entzündlich chronischen Erkankung, ein erhöhter Gehalt eines Akut-Phase-Proteins oder/und eines Regulators der Synthese von Akut-Phase-Proteinen zeigt das Vorliegen einer temporären Akut-Phase-Reaktion an.

Die folgende Darstellung veranschaulicht ein Klassifizierungsmodell, ausgehend von der Bestimmung von Hepcidin und C-reaktivem Protein (CRP).

| | | | |
|---|---|---|---|
| Hepcidin | ⇑ | Hepcidin | ⇑ |
| CRP | normal | CRP | ⇑ |
| Entzündliche chronische Erkrankung keine temporäre Akut-Phase-Reaktion | | Entzündliche chronische Erkrankung mit temporärer Akut-Phase-Reaktion -(Infektionen etc.) | |
| Hepcidin | normal | Hepcidin | normal |
| CRP | normal | CRP | ⇑ |
| "gesund", keine temporäre Akut-Phase-Reaktion, keine entzündliche chronische Erkrankung | | temporäre Akut-Phase-Reaktion, keine entzündliche chronische Erkrankung (Funktionseisen normal) | |
| | | | ⇒ CRP [mg/L] |

Im Hinblick auf die in der Darstellung gezeigte Kombination eines erhöhten Hepcidingehalts mit einem normalen CRP-Wert wird angemerkt, dass das Auftreten einer solchen Kombination grundsätzlich möglich, aber sehr unwahrscheinlich ist.

Auch in dieser Ausführungsform des erfindungsgemäßen Verfahrens können neben Hepcidin und einem Akut-Phase-Protein oder/und einem Regulator der Synthese von Akut-Phase-Proteinen zusätzlich ein oder mehrere Marker verwendet werden. Geeignete Marker sind oben angeführt.

Häufig treten bei der Diagnose von Patienten Fälle auf, in denen sowohl eine Unterscheidung zwischen entzündlichen chronischen Erkrankungen und nicht-entzündlichen chronischen Erkrankungen als auch zwischen entzündlichen chronischen Erkrankungen und Akut-Phase-Reaktionen getroffen werden muss. Vorteilhafterweise ist dies möglich durch Bestimmung der Parameter Hepcidin, Homocystein und einem Akut-Phase-Protein oder/und einem Regulator der Synthese von Akut-Phase-Proteinen.

In einer besonders bevorzugten Ausführungsform des erfindungsgemäßen Verfahrens erfolgt somit die Bestimmung von
(i) Hepcidin,
(ii) Homocystein und
(iii) einem Akut-Phase-Protein oder/und einem Regulator der Synthese von Akut-Phase-Proteinen.

Die Referenzwerte der in dieser Ausführungsform bestimmten Parameter entsprechen denjenigen, welche in den anderen Ausführungsformen des erfindungsgemäßen Verfahrens verwendet werden und oben beschrieben sind.

Bei dieser Ausführungsform kann vorteilhafterweise eine Diagnostik von entzündlich chronischen Erkrankungen, temporären Akut-Phase-Reaktionen oder/und nicht-entzündlichen chronischen Erkrankungen erfolgen. Bevorzugt erfolgt in dieser Ausführungsform eine Klassifizierung in die folgenden Gruppen:
a) weder nicht-entzündliche noch entzündliche chronische Erkrankung, keine Akut-Phase-Reaktion,
b) nicht-entzündliche chronische Erkrankung, (keine entzündliche chronische Erkrankung, keine Akut-Phase-Reaktion),
c) entzündliche chronische Erkrankung und Akut-Phase-Reaktion, (keine nicht-entzündliche chronische Erkrankung),
d) entzündliche chronische Erkrankung (keine Akut-Phase-Reaktion, keine nicht-entzündliche chronische Erkrankung),
e) nicht-entzündliche chronische Erkrankung und Akut-Phase-Reaktion, (keine entzündliche chronische Erkrankung),
f) Akut-Phase-Reaktion (weder nicht-entzündliche noch entzündliche chronische Erkrankung),
g) nicht-entzündliche chronische Erkrankung und entzündliche chronische Erkrankung und Akut-Phase-Reaktion,
h) nicht-entzündliche chronische Erkrankung und entzündliche chronische Erkrankung (keine Akut-Phase-Reaktion).

Ein Patient, der in die Gruppe a) einklassifiziert wird, weist weder eine chronische Erkrankung noch eine Akut-Phase-Reaktion auf und benötigt keine weitere Therapie.

Ein Patient, welcher anhand der bestimmten Werte der Marker i), ii) und iii) in die Gruppe b) einklassifiziert wird, leidet an einer nicht-entzündlichen chronischen Erkrankung. Eine entzündliche chronische Erkrankung oder eine Akut-Phase-Reaktion liegen nicht vor. Mögliche Erkrankungen, welche in Frage kommen, sind beispielsweise B-Vitamin-Mangelerkrankungen des Nervensystems, wie z.B. fumikuläre Spinalerkrankung oder Pseudoencephalitis, Pellagra, neurodegenerative Erkrankungen, wie beispielsweise Alzheimer-Erkrankung, oder vaskuläre Erkankungen, wie z.B. koronare Herzkrankheit, oder arterielle Krankheiten.

Ein Patient, der in der Gruppe c) einklassifiziert wird, leidet an einer entzündlichen chronischen Erkrankung und weist eine Akut-Phase-Reaktion auf. Eine nicht-entzündliche chronische Erkankung liegt bei diesem Patienten nicht vor. Mögliche Erkrankungen, welche in Frage kommen sind z.B. Pankreatitis, Hepatitis, rheumatische Erkankungen, systemischer Lupus erythematodes oder Vaskulitiden.

Ein Patient, der in der Gruppe d) einklassifiziert wird, weist eine entzündliche chronische Erkrankung auf. Eine Akut-Phase-Reaktion oder eine nicht-entzündliche chronische Erkrankung liegt nicht vor. Mögliche Erkankungen, welche in Frage kommen sind beispielsweise chronische Krankheitsanämie (ACD) und Tumorerkrankungen.

Ein Patient, welcher in die Gruppe e) einklassifiziert wird, weist eine nicht-entzündliche chronische Erkrankung und eine Akut-Phase-Reaktion auf. Eine entzündliche chronische Erkrankung liegt nicht vor.

Bei einem Patienten, welcher in die Gruppe f) ein klassifiziert wird, liegt weder eine nicht-entzündliche noch eine entzündliche chronische Erkrankung vor. Der Patient weist lediglich eine Akut-Phase-Reaktion auf.

Ein Patient, der in die Gruppe g) einklassifiziert wird, weist sowohl eine nicht-entzündliche chronische Erkrankung als auch eine entzündliche chronische Erkrankung und eine Akut-Phase-Reaktion auf.

Ein Patient, welcher in die Gruppe h) einklassifiziert wird, weist sowohl eine nicht-entzündliche als auch eine entzündliche chronische Erkrankung, jedoch keine Akut-Phase-Reaktion auf.

Bei allen Ausführungsformen des erfindungsgemäßen Verfahrens können zu den genannten Parametern ein oder mehrere weitere Marker bestimmt werden. Bevorzugt werden beispielsweise Holotranscobalamin II, Methylmalonsäure, Cystathionin, Akut-Phase-Proteine, Regulatoren der Synthese von Akut-Phase-Proteinen, Transferin, löslicher Transferinrezeptor (sTfR), Ferritin, Creatinin, Hämoglobin, Blutglucose, Triglyceride, Cholesterin, Zink, Protoporphyrin, Myoglobin, Hämosiderin, Katalase, Peroxidase und Cytochrom bestimmt. Zudem können auch andere, im Fachgebiet eingesetzte Marker verwendet werden. Durch eine Kombination der erfindungsgemäßen Marker mit weiteren Markern ist es vorteilhafterweise möglich, ein sehr breites Spektrum unterschiedlichster Erkrankungen in einem Diagnoseschritt zu identifizieren.

Das erfindungsgemäße Verfahren ermöglicht vorteilhafterweise auch eine Differenzialdiagnostik. Eine Differenzialdiagnostik ist ausgerichtet auf die Abgrenzung und Identifizierung einer oder mehrerer bestimmter Krankheiten innerhalb einer Gruppe symptomatisch ähnlicher bzw. zum Teil sogar übereinstimmender Krankheiten. Überraschenderweise kann mit dem erfindungsgemäßen Verfahren eine Abgrenzung bzw. Identifizierung von entzündlichen chronischen Erkrankungen, nicht-entzündlichen chronischen Erkrankungen und Akut-Phase-Reaktionen, welche oft sehr ähnliche Symptome aufweisen, erfolgen.

Des Weiteren kann das Verfahren gemäß der vorliegenden Erfindung in Kombination mit anderen Diagnostikverfahren bzw. Differenzialdiagnostikverfahren verwendet werden. Besonders bevorzugt ist beispielsweise die Kombination des erfindungsgemäßen Verfahrens mit einem Test zur Bestimmung von Eisenstoffwechselstörungen oder/und Vitaminstörungen. Hierbei werden bevorzugt Störungen des Vitamin B6, Vitamin B12 und Folsäurehaushalts bestimmt. Somit kann vorteilhafterweise nicht nur die Krankheit selbst, sondern in vielen Fällen auch die Ursache der Erkrankung, beispielsweise ein Vitaminmangel, in einem Diagnoseschritt ermittelt werden.

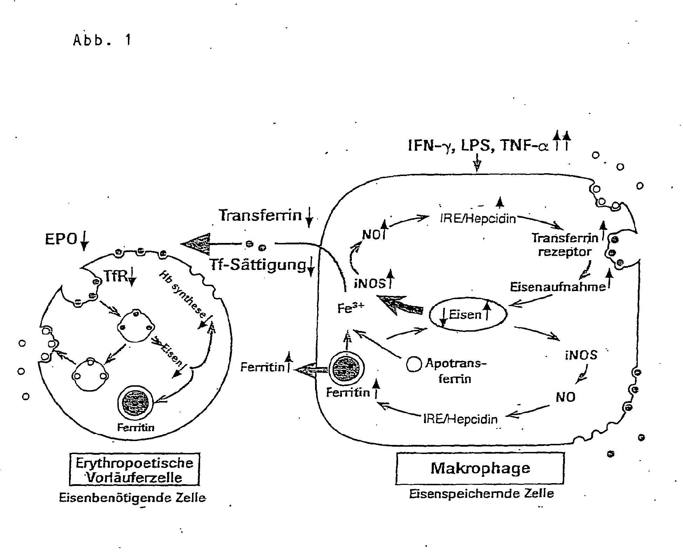

Die vorliegende Erfindung wird weiterhin durch die Abbildungen 1 bis 3 veranschaulicht.
Abbildung 1 zeigt ein Modell der Autoregulation von Eisenstoffwechsel und NO/NOS-Zyklus in aktivierten Monozyten/Makrophagen und der Versorgung einer Eisen benötigenden Zelle (Weiß, G. et al., 1997) Die Abkürzungen in Abb. 1 sind wie folgt: IFN-γ: Interferon γ, iNOS: induzierte Stickoxydsynthase, IRE: auf Eisen reagierendes Element, IRE/Hepcidin: hochaffine Bindung von Hepcidin an IRES, LPS: Lipopolysaccharid, TNF-α: Tumor-Nekrose-Faktor α, | und | zeigen Anstieg oder Abnahme zellulärer Reaktionen bei der Versorgung einer Eisen-benötigenden Zelle an. Aus Abbildung 1 ist ersichtlich, dass durch Hochregulierung von Hepcidin die Transferrinsynthese gedrosselt wird und die Transferrinsättigung erniedrigt wird. In Makrophagen bewirkt eine hohe Eisenkonzentration eine Erhöhung der induzierten Stickoxidsynthetase (iNOS). Diese induziert die Cytokinproduktion, was chronische Entzündungen auslösen kann.

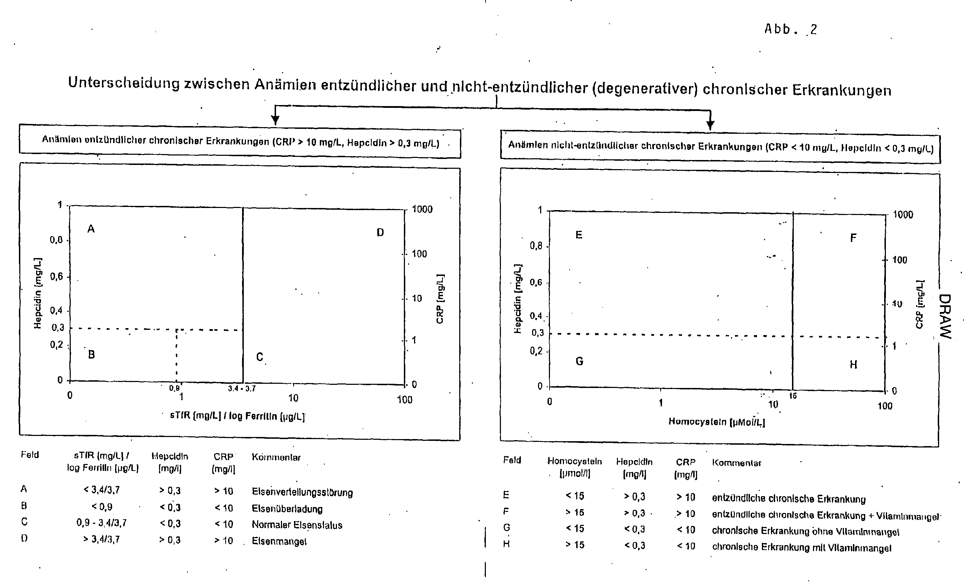

Abbildung 2 zeigt eine schematische Darstellung der Unterscheidung zwischen Anämien entzündlicher und nicht-entzündlicher chronischer Erkrankungen.

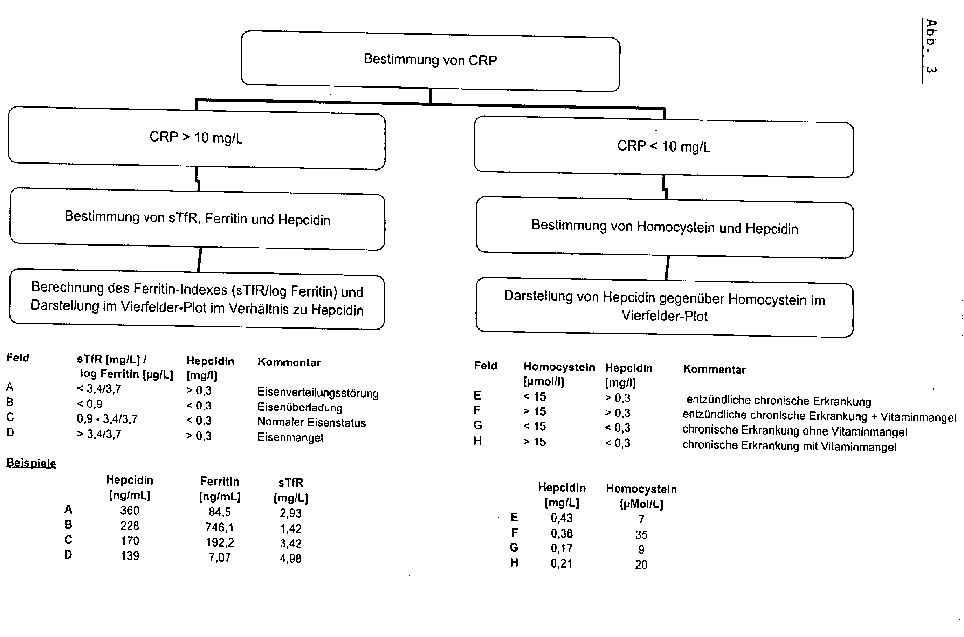

Abbildung 3 zeigt eine Vorgehensweise zur Differenzialdiagnostik unter Verwendung verschiedener Marker, darunter CRP, sTfR, Ferritin, Hepcidin und Homocystein. Die Beispiele A bis H veranschaulichen verschiedene durch die erfindungsgemäße Vorgehensweise unterscheidbare Krankheitsbilder. Die Bestimmung von CRP, sTfR und Ferritin erfolgte über homogene Immunoassays (Tina-quant®, Roche Diagnostics), die von Homocystein über einen homogenen Immunoassay der Firma Axis-Shields (Oslo, Norwegen) und die von Hepcidin über einen ELISA-Test (Hepcidin Prohormone ELISA der Firma DRG, Marburg, Deutschland).

### Beispiele

### Beispiel 1

Von Patienten A bis H mit den in Fig. 3 unter "Kommentar" angegebenen Krankheitsbildern wurden die Marker CRP, sTfR, Ferritin, Hepcidin und Homocystein bestimmt. Die erhaltenen Werte sind in Fig. 3 angegeben.

### Beispiel 2

Von 283 Patienten mit entzündlichen chronischen Erkrankungen wurden die Marker Hepcidin, CRP, sTfR und Ferritin bestimmt. Die Ergebnisse sind in Tabelle 1 angegeben.

**Tabelle 1**

| Hepcidin-Prohormon, ein neuer Marker in der Diagnose von Eisenstoffwechseistörungen | | | | | | |
|---|---|---|---|---|---|---|
| **Gruppe** | **Alter [Jahre]** | **n** | **Mittel** | | | |
| | | | **Hepcidin* [µg/L]** | **CRP [mg/L]** | **sTfR [mg/L]** | **Ferritin [µg/L]** |
| Frauen | 50-59 | 48 | 141 | 1.7 | 3.2 | 70 |
| | 60-69 | 64 | 125 | 1.7 | 3.2 | 90 |
| | 70-80 | 28 | 121 | 2.0 | 3.2 | 89 |
| Hormontherapie | | 37 | 123 | 1.8 | 2.8 | 94 |
| Keine Hormontherapie | | 105 | 136 | 1.9 | 3.3 | 77 |
| Männer | 50-59 | 56 | 140 | 1.4 | 3.0 | 166 |
| | 60-69 | 60 | 139 | 1.5 | 3.3 | 188 |
| | 70-80 | 28 | 138 | 2.2 | 3.3 | 198 |
| *Erwarteter Hepcidin-Normalbereich: < 100-300 µg/l | | | | | | |

Die Ergebnisse zeigen, dass Hepcidin ein Marker der Menge an zirkulierendem Eisen ist. Wenn die Menge an zirkulierendem Eisen zu hoch ist (sTfR < 4 mg/l; Ferritin > 100 µg/l), wird Hepcidin freigesetzt. (> 300 µg/l). Wenn die Menge an zirkulierendem Eisen zu gering ist (sTfR > 4 mg/l, Ferritin < 30 µg/l), wird die Hepcidin-Expression gestoppt.

Die folgenden Grenzwerte wurden gefunden:

| | |
|---|---|
| Hepcidin, erwarteter Normalbereich | < 100-300 µg/l |
| sTfR/log Ferritin bei Patienten mit Eisenmangel (w,m) | 0,9 |
| sTfR/log Ferritin bei Patienten mit Eisenüberladung (w,m) | 3,7 und 3,4 |
| sTfR bei Patienten mit Eisenmangel (w,m) | 4,4 und 5 mg/l |
| Ferritin bei Patienten mit Eisenmangel (w,m) | 15 und 30 µg/l |
| Ferritin bei Patienten mit Eisenüberladung (w,m) | 150 und 400 µg/l |
| CRP bei Patienten mit anhaltender Akut-Phase-Reaktion | > 5 mg/l |

## Patentansprüche

1. Verfahren zum Nachweis von chronischen Erkrankungen, umfassend die Bestimmung von
i) Hepcidin und
ii) Homocystein,
worin entzündliche oder/und nicht-entzündliche chronische Erkrankungen nachgewiesen werden und eine Klassifizierung in die folgenden Gruppen erfolgt:
a) keine chronische Erkrankung,
b) entzündliche chronische Erkrankung,
c) nicht-entzündliche chronische Erkrankung,
d) sowohl entzündliche chronische Erkrankung als auch nicht-entzündliche chronische Erkrankung.

2. Verfahren zum Nachweis von entzündlichen chronischen Erkrankungen oder/und Akut-Phase-Reaktionen, umfassend die Bestimmung von
i) Hepcidin und
iii) einem Akut-Phase-Protein oder/und einem Regulator der Synthese von Akut-Phase-Proteinen,
**dadurch gekennzeichnet,**
**dass** eine Klassifizierung in die folgenden Gruppen erfolgt:
a) keine Akut-Phase-Reaktion, keine entzündliche chronische Erkrankung,
b) entzündliche chronische Erkrankung mit Akut-Phase-Reaktion, und
c) Akut-Phase-Reaktion, keine entzündliche chronische Erkrankung und
d) entzündliche chronische Erkrankung, keine Akut-Phase-Reaktion.

3. Verfahren nach Anspruch 2, worin das Akut-Phase-Protein C-reaktives Protein ist.

4. Verfahren nach einem der Ansprüche 1 bis 3, umfassend die Bestimmung von
i) Hepcidin,
ii) Homocystein und
iii) einem Akut-Phase-Protein oder/und einem Regulator der Synthese von Akut-Phase-Proteinen.

5. Verfahren nach Anspruch 4, worin eine Klassifizierung in die folgenden Gruppen erfolgt:
a) weder nicht-entzündliche noch entzündliche chronische Erkrankung, keine Akut-Phase-Reaktion,
b) nicht-entzündliche chronische Erkrankung, (keine entzündliche chronische Erkrankung, keine Akut-Phase-Reaktion),
c) entzündliche chronische Erkrankung und Akut-Phase-Reaktion, (keine nicht-entzündliche chronische Erkrankung),
d) entzündliche chronische Erkrankung (keine Akut-Phase-Reaktion, keine nicht-entzündliche chronische Erkrankung),
e) nicht-entzündliche chronische Erkrankung und Akut-Phase-Reaktion, (keine entzündliche chronische Erkrankung),
f) Akut-Phase-Reaktion (weder nicht-entzündliche noch entzündliche chronische Erkrankung),
g) nicht-entzündliche chronische Erkrankung und entzündliche chronische Erkrankung und Akut-Phase-Reaktion,
h) nicht-entzündliche chronische Erkrankung und entzündliche chronische Erkrankung (keine Akut-Phase-Reaktion).

6. Verfahren nach einem der Ansprüche 1 bis 5,
**dadurch gekennzeichnet,**
**dass** ein oder mehrere weitere Marker bestimmt werden, ausgewählt aus Holotranscobalamin II, Methylmalonsäure, Cystathionin, Akut-Phase-Proteinen, Regulatoren der Synthese von Akut-Phase-Proteinen, Transferin, löslichem Transferinrezeptor (sTfR), Ferritin, Creatinin, Hämoglobin, Blutglucose, Triglyceride, Cholesterin, Zink, Protoporphyrin, Myoglobin, Hämosiderin, Katalase, Peroxidase und Cytochrom.

7. Verfahren nach einem der Ansprüche 1 bis 6,
**dadurch gekennzeichnet,**
**dass** eine Differenzialdiagnostik durchgeführt wird.

8. Verwendung eines Verfahrens nach einem der Ansprüche 1 bis 7 in Kombination mit einem Test zur Bestimmung von Eisenstoffwechselstörungen oder/und Vitaminstörungen.

## Claims

1. A method of detecting chronic disorders, comprising the determination of
i) hepcidin and
ii) homocysteine,
wherein inflammatory and/or non-inflammatory chronic disorders are detected and a classification into the following groups takes place:
a) no chronic disorder,
b) inflammatory chronic disorder,
c) non-inflammatory chronic disorder,
d) both inflammatory chronic disorder and non-inflammatory chronic disorder.

2. A method of detecting inflammatory chronic disorders and/or acute-phase reactions, comprising the determination of
i) hepcidin and
iii) an acute-phase protein and/or a regulator of the synthesis of acute-phase proteins,
**characterised in**
**that** a classification into the following groups takes place:
a) no acute-phase reaction, no inflammatory chronic disorder,
b) inflammatory chronic disorder with acute-phase reaction, and
c) acute-phase reaction, no inflammatory chronic disorder and
d) inflammatory chronic disorder, no acute-phase reaction.

3. A method according to Claim 2, wherein the acute-phase protein is C-reactive protein.

4. A method according to one of Claims 1 to 3, comprising the determination of
i) hepcidin,
ii) homocysteine and
iii) an acute-phase protein and/or a regulator of the synthesis of acute-phase proteins.

5. A method according to Claim 4, wherein a classification into the following groups takes place:
a) neither non-inflammatory nor inflammatory chronic disorder, no acute-phase reaction,
b) non-inflammatory chronic disorder, (no inflammatory chronic disorder, no acute-phase reaction),
c) inflammatory chronic disorder and acute-phase reaction, (no non-inflammatory chronic disorder),
d) inflammatory chronic disorder (no acute-phase reaction, no non-inflammatory chronic disorder),
e) non-inflammatory chronic disorder and acute-phase reaction, (no inflammatory chronic disorder),
f) acute-phase reaction (neither non-inflammatory nor inflammatory chronic disorder),
g) non-inflammatory chronic disorder and inflammatory chronic disorder and acute-phase reaction,
h) non-inflammatory chronic disorder and inflammatory chronic disorder (no acute-phase reaction).

6. A method according to one of Claims 1 to 5,
**characterised in**
**that** one or more further markers are determined, selected from holotranscobalamin II, methylmalonic acid, cystathionine, acute-phase proteins, regulators of the synthesis of acute-phase proteins, transferrin, soluble transferrin receptor (sTfR), ferritin, creatinine, haemoglobin, blood glucose, triglycerides, cholesterol, zinc, protoporphyrin, myoglobin, haemosiderin, catalase, peroxidase and cytochrome,

7. A method according to one of Claims 1 to 6,
**characterised in**
**that** a differential diagnosis is carried out.

8. Use of a method according to one of Claims 1 to 7 in combination with a test for determining iron metabolism disorders and/or vitamin disorders.

## Revendications

1. Procédé de détection de maladies chroniques, comprenant la détermination de
i) l'hepcidine et
ii) l'homocystéine,
dans lequel des maladies chroniques inflammatoires et/ou non inflammatoires sont détectées et une classification s'effectue dans les groupes suivants :
a) pas de maladie chronique
b) maladie chronique inflammatoire,
c) maladie chronique non inflammatoire,
d) à la fois maladie chronique inflammatoire et également maladie chronique non inflammatoire.

2. Procédé de détection de maladies chroniques inflammatoires et/ou de réactions en phase aiguë, comprenant la détermination de
i) l'hepcidine et
iii) d'une protéine de phase aiguë et/ou d'un régulateur de la synthèse des protéines de phase aiguë,
**caractérisé en ce que**
une classification s'effectue dans les groupes suivants :
a) pas de réaction de phase aiguë, pas de maladie chronique inflammatoire,
b) maladie chronique inflammatoire avec réaction de phase aiguë, et
c) réaction de phase aiguë, pas de maladie chronique inflammatoire et
d) maladie chronique inflammatoire, pas de réaction de phase aiguë.

3. Procédé selon la revendication 2, dans lequel la protéine de phase aiguë est une protéine C réactive.

4. Procédé selon l'une des revendications 1 à 3, comprenant la détermination de
i) l'hepcidine,
ii) l'homocystéine et
iii) d'une protéine de phase aiguë et/ou d'un régulateur de la synthèse des protéines de phase aiguë.

5. Procédé selon la revendication 4, dans lequel une classification s'effectue dans les groupes suivants :
a) ni maladie chronique non inflammatoire, ni maladie chronique inflammatoire, pas de réaction de phase aiguë,
b) maladie chronique non inflammatoire (pas de maladie chronique inflammatoire, pas de réaction de phase aiguë),
c) maladie chronique inflammatoire et réaction de phase aiguë, (pas de maladie chronique non inflammatoire),
d) maladie chronique inflammatoire (pas de réaction de phase aiguë, pas de maladie chronique non inflammatoire),
e) maladie chronique non inflammatoire et réaction de phase aiguë, (pas de maladie chronique inflammatoire),
f) réaction de phase aiguë (ni maladie chronique non inflammatoire, ni maladie chronique inflammatoire),
g) maladie chronique non inflammatoire et maladie chronique inflammatoire et réaction de phase aiguë,
h) maladie chronique non inflammatoire et maladie chronique inflammatoire (pas de réaction de phase aiguë).

6. Procédé selon l'une des revendications 1 à 5,
**caractérisé en ce que**
un ou plusieurs autres marqueurs sont déterminés, choisis parmi l'holotranscobalamine II, l'acide méthylmalonique, la cystathionine, les protéines de phase aiguë, les régulateurs de la synthèse des protéines de phase aiguë, la transférine, le récepteur de la transférine soluble (sTfR), la ferritine, la créatinine, l'hémoglobine, le glucose sanguin, les triglycérides, le cholestérol, le zinc, la protoporphyrine, la myoglobine, l'hémosidérine, la catalase, la peroxydase et le cytochrome.

7. Procédé selon l'une des revendications 1 à 6,
**caractérisé en ce que**
un diagnostic différentiel est réalisé.

8. Utilisation d'un procédé selon l'une des revendications 1 à 7, en combinaison avec un test pour déterminer les troubles du métabolisme du fer et/ou les troubles vitaminiques.


### SEQUENZPROTOKOLL

<110> F. Hoffmann-La Roche AG
<120> Differenzialdiagnostik mit Hepcidin
<130> 30966P EP
<140> 04 025 211.6
   <141> 2004-10-22
<150> DE 103 49 124.4
   <151> 2003-10-22
<160> 3
<170> PatentIn Ver. 2.1
<210> 1
   <211> 20
   <212> PRT
   <213> Human
<400> 1
   Ile Cys Ile Phe Cys Cys Gly Cys Cys His Arg Ser Lys Cys Gly Met
    1               5                  10                  15
   Cys Cys Lys Thr
               20
<210> 2
   <211> 22
   <212> PRT
   <213> Human
<400> 2
   Phe Pro Ile Cys Ile Phe Cys Cys Gly Cys Cys His Arg Ser Lys Cys
   1 5 10 15
   Gly Met Cys Cys Lys Thr
   20
<210> 3
   <211> 25
   <212> PRT
   <213> Human
<400> 3
   Asp Thr His Phe Pro Ile Cys Ile Phe Cys Cys Gly Cys Cys His Arg
   1 5 10 15
   Ser Lys Cys Gly Met Cys Cys Lys Thr
   20 25